# EUROPEAN PATENT APPLICATION

(11) **EP 3 933 393 A1**
(43) Date of publication of application: **05.01.2022**
(21) Application number: 20783031.6
(22) Date of filing: 23.03.2020
(51) Int. Cl.: G01N 27/26, G01N 27/28, G01N 27/327, G01N 27/416, C12Q 1/04

(54) **MEASUREMENT DEVICE, MEASUREMENT METHOD, PROGRAM, AND BIOSENSOR**

(30) Priority: 02.04.2019 JP 2019070305
(71) Applicant: National Institute for Materials Science, Tsukuba-shi, Ibaraki 305-0047 (JP)
(72) Inventor: OKAMOTO, Akihiro, Tsukuba-shi, Ibaraki 305-0047 (JP)
(74) Representative: Markfort, Iris-Anne Lucie
(86) International application number: PCT/JP2020/012673
(87) International publication number: WO 2020/203404

(57) **Abstract**

[Object] To provide a measurement apparatus capable of easily acquiring microbiota information regarding a specimen, and also provide a measurement method and a program.

[Solving Means] A measurement apparatus includes: a voltage applying unit that applies a voltage between at least two electrodes disposed so as to come into contact with a complex in which a specimen including a microorganism and a medium including a substrate are in contact with each other; a measuring unit that measures a response when the voltage is applied; a storage unit in which a classifier is stored; and an analysis output unit that applies the substrate and the response to the classifier, and outputs microbiota information regarding the specimen, in which the classifier is a classifier which has been pre-learned by using learning data including known microbiota information regarding a learning specimen, a substrate, and a response to be obtained so as to output, when the substrate used for measurement and the obtained response are input, microbiota information regarding a specimen to be measured.

## Description

### Technical Field

The present invention relates to a measurement apparatus, a measurement method, a program, and a biosensor.

### Background Art

A technology for acquiring microbiota information in a specimen containing a microorganism has been known.

Patent Literature 1 describes, for example, a method of analyzing fungal flora with a T-RFLP (Terminal Restriction Fragment Length Polymorphisms) method using DNA (Deoxyribonucleic Acid) extracted from a human gingival margin and/or submarginal plaque.

### Citation List

### Patent Literature

Patent Literature 1: Japanese Patent Application Laid-open No. 2011-193810

### Disclosure of Invention

### Technical Problem

The method described in Patent Literature 1 extracts DNA from a specimen and performs microbiota analysis, and the operation thereof has been complicated. Further, it has been difficult to easily acquire microbiota information because specialized knowledge is necessary.

In this regard, an object of the present invention is to provide a measurement apparatus capable of easily acquiring microbiota information regarding a specimen, and also provide a measurement method, a program, and a biosensor.

### Solution to Problem

As a result of intensive studies to achieve the object described above, the present inventors have found that the object described above can be achieved by the following configuration.
[1] A measurement apparatus, including:
   a voltage applying unit that applies a voltage between at least two electrodes disposed so as to come into contact with a complex in which a specimen including a microorganism and a medium including a substrate are in contact with each other;
   a measuring unit that measures a response when the voltage is applied;
   a storage unit in which a classifier is stored; and
   an analysis output unit that applies the substrate and the response to the classifier, and outputs microbiota information regarding the specimen, in which
   the classifier is a classifier which has been pre-learned by using learning data including known microbiota information regarding a learning specimen, a substrate, and a response to be obtained so as to output, when the substrate used for measurement and the obtained response are input, microbiota information regarding a specimen to be measured.
[2] The measurement apparatus according to [1], in which
   one of the electrodes is at least one selected from the group consisting of a reference electrode and a counter electrode.
[3] The measurement apparatus according to [1] or [2], in which
   the voltage applying unit applies a predetermined voltage.
[4] The measurement apparatus according to [1] or [2], in which
   the voltage applying unit applies a sweep voltage.
[5] The measurement apparatus according to any one of [1] to [4], in which
   the specimen includes saliva.
[6] The measurement apparatus according to any one of [1] to [5], in which
   the specimen includes periodontal disease bacteria.
[7] The measurement apparatus according to any one of [1] to [6], in which
   the medium is a solid electrolyte.
[8] The measurement apparatus according to [7], in which
   the substrate included in the solid electrolyte is the substrate included in the learning data.
[9] A measurement method, including:
   applying a voltage between at least two electrodes disposed so as to come into contact with a complex in which a specimen including a microorganism and a medium including a substrate are in contact with each other, and measuring a response; and
   applying the substrate and the response to a classifier, and acquiring microbiota information regarding the specimen, in which
   the classifier is a classifier which has been pre-learned by using learning data including known microbiota information regarding a learning specimen, a substrate, and a response to be obtained so as to output, when the substrate used for measurement and the obtained response are input, microbiota information regarding a specimen to be measured.
[10] A program that causes a computer to execute the steps of:
   applying a voltage between at least two electrodes disposed so as to come into contact with a complex in which a specimen including a microorganism and a medium including a substrate are in contact with each other, and measuring a response; and
   applying the substrate and the response to a classifier, and acquiring microbiota information regarding the specimen, in which
   the classifier is a classifier which has been pre-learned by using learning data including known microbiota information regarding a learning specimen, a substrate, and a response to be obtained so as to output, when the substrate used for measurement and the obtained response are input, information for speculating microbiota of a specimen to be measured.
[11] A biosensor, including
   a support; and
   a plurality of cells disposed on the support, in which
   each of the cells includes a solid electrolyte and at least two electrodes disposed so as to come into contact with the solid electrolyte, and
   the solid electrolytes include substrates different from each other.

### Advantageous Effects of Invention

In accordance with the present invention, it is possible to provide a measurement apparatus capable of easily acquiring microbiota information regarding a specimen. Further, in accordance with the present invention, it is possible to provide also a measurement method, a program, and a biosensor.

### Brief Description of Drawings

[Fig. 1] Fig. 1 is a perspective view showing an example of a measurement apparatus according to an embodiment of the present invention and a biosensor that is loaded in the measurement apparatus for use.
[Fig. 2] Fig. 2 is an exploded perspective view of a biosensor that is loaded in the measurement apparatus for use.
[Fig. 3] Fig. 3 is an exploded perspective view of a biosensor that is loaded in the measurement apparatus for use.
[Fig. 4] Fig. 4 is a functional block diagram of the measurement apparatus according to the embodiment of the present invention.
[Fig. 5] Fig. 5 is a flowchart in which a control unit of the measurement apparatus performs measurement in accordance with a program stored in a storage unit.
[Fig. 6] Fig. 6 shows an electrochemical response of a solution containing a specimen that contains PG (Porphyromonas gingivalis) bacteria.
[Fig. 7] Fig. 7 shows an electrochemical response of a solution containing a specimen that contains AA(Aggregatibacter actinomycetemcomitans) bacteria.
[Fig. 8] Fig. 8 is a diagram showing an example of a four-layer deep neural network.
[Fig. 9] Fig. 9 shows an example of training data to be learned by a neural network.
[Fig. 10] Fig. 10 shows an example of training data to be learned by a neural network.
[Fig. 11] Fig. 11 is a sequence diagram showing processing executed in the measurement apparatus according to the embodiment of the present invention.
[Fig. 12] Fig. 12 shows an example of a substrate list.
[Fig. 13] Fig. 13 shows an example of a substrate inquiry screen display.
[Fig. 14] Fig. 14 shows an example of a display screen after transition of the substrate inquiry screen display.
[Fig. 15] Fig. 15 shows an example of a display screen after transition of the substrate inquiry screen display.
[Fig. 16] Fig. 16 shows an example of a display screen after transition of the substrate inquiry screen display.
[Fig. 17] Fig. 17 is a perspective view of a biosensor according to a different embodiment, which is loaded in the measurement apparatus according to the embodiment of the present invention for use.
[Fig. 18] Fig. 18 is an exploded perspective view of a biosensor according to the different embodiment.
[Fig. 19] Fig. 19 is a schematic diagram showing an electrode of the biosensor according to the different embodiment.

### Mode(s) for Carrying Out the Invention

Hereinafter, the present invention will be described in detail.

The description of the configuration requirements described below is made on the basis of the typical embodiment of the present invention in some cases, but the present invention is not limited to such an embodiment.

Note that in the present specification, the numerical value range represented by using "to" means the range including the numerical values described before and after "to" as the lower limit value and the upper limit value.

### [Measurement apparatus]

A measurement apparatus according to an embodiment of the present invention is a measurement apparatus including: a voltage applying unit that applies a voltage between at least two electrodes disposed so as to come into contact with a complex in which a specimen including a microorganism and a medium including a substrate are in contact with each other; a measuring unit that measures a response when the voltage is applied; a storage unit in which a classifier is stored; and an analysis output unit that applies the substrate and the response to the classifier, and outputs microbiota information regarding the specimen, in which the classifier is a classified which has been pre-learned by using learning data including known microbiota information regarding a learning specimen, a substrate, and a response to be obtained so as to output, when the substrate used for measurement and the obtained response are input, microbiota information regarding a specimen to be measured.

### [Apparatus configuration]

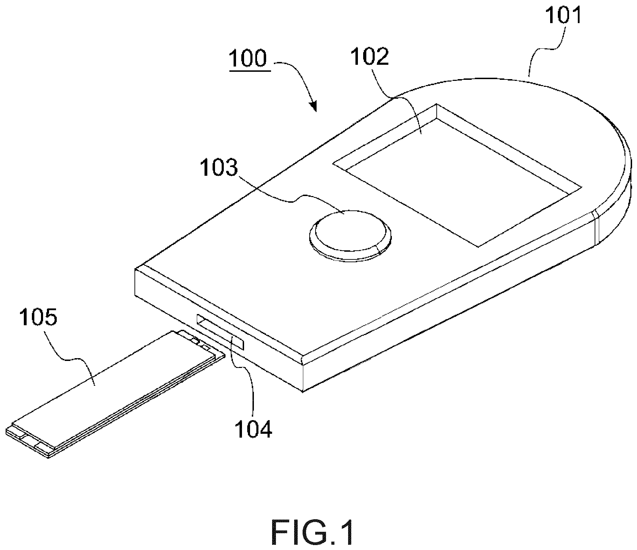

Fig. 1 is a perspective view showing an example of a measurement apparatus according to an embodiment of the present invention (hereinafter, referred to also as "this measurement apparatus") and a biosensor that is loaded in the measurement apparatus for use.

A measurement apparatus 100 includes a body 101, a display unit 102, an operation button 103, and an insertion port 104 for loading the biosensor 105. A user loads the biosensor 105 via the insertion port 104, and can perform desired measurement by operating the operation button 103 in accordance with the display of the display unit 102.

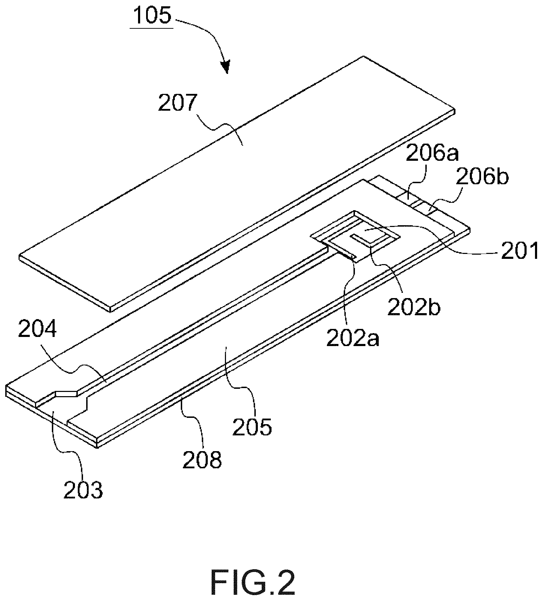

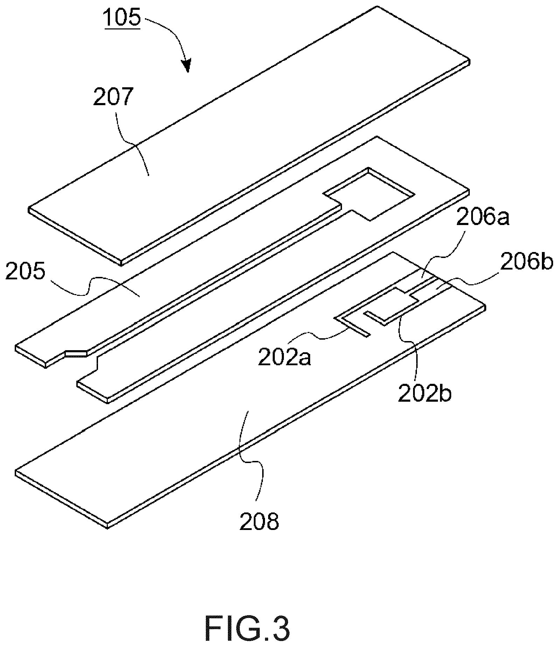

Fig. 2 and Fig. 3 are each an exploded perspective view of the biosensor 105. The biosensor 105 is typically disposable, and it is possible to perform more accurate measurement by using a new biosensor 105 for each measurement.

The biosensor 105 includes a cell 201 that is a region for introducing a specimen, a medium, and/or a complex (hereinafter, referred to also as "solution"), and a pair of electrodes 202 (including a first electrode 202a and a second electrode 202b) disposed so as to come into contact with a complex introduced in the cell.

The cell 201 is defined by a support 208, a capillary substrate 205 disposed on the support 208, and a cover 207. A solution is introduced from an introduction port 203, flows in a conduit part 204, and then is introduced in the cell 201. When being introduced in the cell 201, the solution comes into contact with the electrodes 202. Note that Fig. 2 shows the support 208 and the capillary substrate 205 in the unseparated state, and Fig. 3 shows the support 208 and the capillary substrate 205 in the separated state.

The first electrode 202a and the second electrode 202b are electrically connected to electrode pads 206a and 206b, respectively. When the biosensor 105 is loaded (inserted) in the body 101 from the insertion port 104 shown in Fig. 1, the electrode pads 206a and 206b are electrically connected to a circuit substrate disposed in the body 101 via a connector part disposed in the body 101, and a voltage is applied between the pair of electrodes 202 by a voltage applying unit controlled by the control unit described below, making it possible to measure a response to the applied voltage from a complex.

Note that the response is, for example, an electrochemical response such as a current value at a certain measurement time and a change in the current value over time, and may include, for example, a temperature of the complex in addition to the above.

The cell described above may be airtightly configured. In the case where the cell is airtightly configured, measurement with higher sensitivity can be performed in some cases. The method of airtightly configurating the cell is not particularly limited, and a known method can be applied. Examples of such a method include a method of using a cell with a lid, which includes a cell and a lid portion covering an opening of the cell.

Further, the thicknesses of the support 208, the capillary substrate 205, and the cover 207 are not particularly limited and can be appropriately selected. From the viewpoint of ease of handling, the favorable thicknesses are typically 0.1 µm to 10 mm.

After the biosensor 105 is loaded in the body 101, an operator operates the operation button 103 and then measurement is started. The solution identification number of the solution, measurement conditions, measurement results, and the like are displayed on the display unit 102.

In this measurement apparatus, the biosensor 105 can be attached and detached, the biosensor 105 can be replaced with a new one for each measurement, contamination for each measurement is suppressed, and measurement with higher accuracy can be performed.

Note that the measurement apparatus according to the embodiment of the present invention is not limited to the above, and the biosensor 105 and the measurement apparatus 100 may be integrated. In the case where the biosensor and the body are integrated, the measurement apparatus can be more easily produced because the structure of the measurement apparatus is simpler.

This measurement apparatus includes the operation button 103. However, the measurement apparatus according to the embodiment of the present invention is not limited to the above, and does not necessarily need to include the operation button 103. In the case where the measurement apparatus does not include the operation button 103, the display unit 102 may include a touch panel and an instruction to start measurement by an operator may be received via a GUI (Graphical User Interface) by screen display of the display unit 102. Further, the measurement apparatus does not include the operation button 103, and measurement may be started automatically when the biosensor 105 is loaded in the insertion port 104 of the body 101.

Since this measurement apparatus includes the display unit 102, a series of steps from setting of measurement conditions to displaying of measurement results can be performed by a single apparatus, and microbiota information can be easily acquired on-site (in other words, microbiota analysis can be performed). Note that the display unit described above only needs to be a liquid crystal display, an organic EL (Electro Luminescence), or the like, and may further have a function as a touch panel.

Further, this measurement apparatus includes an air conditioner (not shown) in the body 101. As the air conditioner, a heater or the like can be used. Since this measurement apparatus 100 includes an air conditioner, the measurement temperature can be kept constant and measurement results with higher accuracy can be achieved.

The respective units of this measurement apparatus will be described below in detail.

### <Electrode>

In this measurement apparatus, the first electrode 202a is a working electrode, and the second electrode 202b is a counter electrode. However, the measurement apparatus according to the embodiment of the present invention is not limited to the above. The second electrode 202b may be a reference electrode. Further, in the biosensor 105, still another electrode (third electrode) may be in contact with a solution. In this case, the third electrode is favorably a reference electrode. That is, the first electrode 202a, the second electrode 202b, and the third electrode may respectively be a working electrode, a counter electrode, and a reference electrode, or a working electrode and two reference electrodes. Note that in the measurement apparatus including a reference electrode, an electrode potential can be measured, and a measurement apparatus having more excellent effects of the present invention can be obtained.

In Fig. 2, a pair of electrodes combined in a key shape is shown. However, the shapes of the electrodes are not particularly limited thereto, and the electrodes may be comb electrodes (interdigit electrode). These electrodes can be produced by a known method. For example, the electrodes can be disposed in a pattern on a support by a photolithography method, a plating method, a printing method, or the like. The distance between the electrodes, and the like are not particularly limited, and only needs to be a distance known as that of an electrochemical cell. In particular, the area of the electrode in contact with a solution is favorably 1 cm² or less because electrochemical measurement can be performed with favorable sensitivity even with a smaller solution (specifically, 0.001 to 5 ml).

The material of the electrode is not particularly limited, and a known electrode material can be used. Examples of the electrode material include carbon, gold, platinum, silver, molybdenum, cobalt, nickel, palladium, and ruthenium. Alternatively, indium tin oxide or the like may be used, and a known material for an electrode can be used.

Note that as the reference electrode, a known reference electrode can be used. For example, a silver/silver chloride electrode can be used. Further, as the counter electrode, a known counter electrode can be used.

### <Cell>

The cell 201 is a region provided in the biosensor 105 for introducing a complex in which a specimen containing a microorganism and a medium containing a substrate come into contact with each other, and the cell 201 is defined by the support 208, the capillary substrate 205, and the cover 207 as shown in Fig. 2.

The cell is not limited to the above, and may include a container having an opening and at least a pair of electrodes disposed in the container.

In any case, the cell is favorably formed of an insulating material.

Examples of the insulating material include an organic material, an inorganic material, a composite thereof, and more specifically, a resin, paper, and glass.

Examples of the resin include a thermoplastic resin such as polyetherimide (PEI), polyethylene terephthalate (PET), and polyethylene(PE); and a thermosetting resin such as a polyimide resin and an epoxy resin.

As the insulating material, for example, glass, paper, or the like may be used.

The size of the cell 201 is not particularly limited. However, the size of the cell 201 can be appropriately selected in accordance with the amount of the solution to be measured, and favorably has the capacity of approximately 0.0001 to 5 ml.

Further, the cell may be configured to be airtight. In the case where the cell is configured to be airtight, measurement with higher sensitivity can be performed in some cases. The method of configuring the cell to be airtight is not particularly limited, and a known method can be applied. Examples of such a method include a method of using a cell with a lid, which includes a cell and a lid portion covering an opening of the cell.

### [Function]

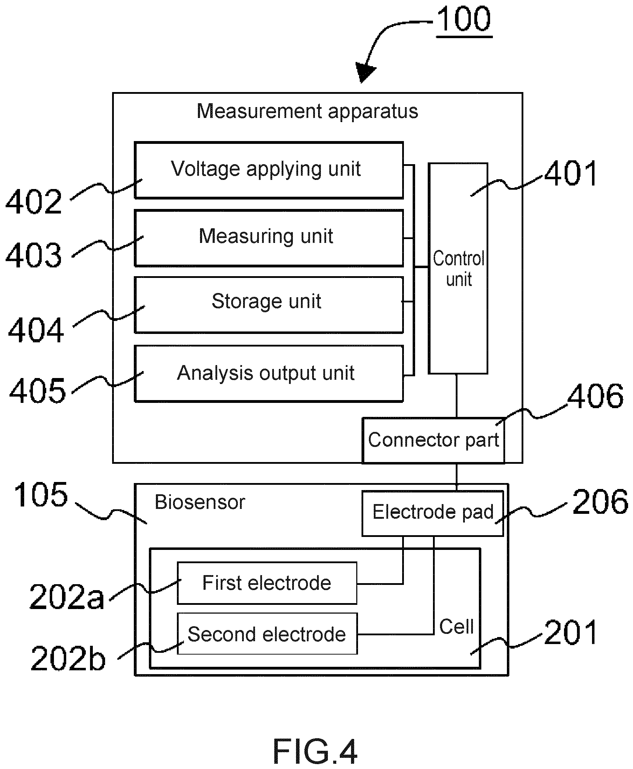

Fig. 4 is a functional block diagram of this measurement apparatus. Fig. 4 shows the state where the biosensor 105 including the cell 201 in which a solution has been introduced is already loaded in the body of the measurement apparatus and the first electrode 202a and the second electrode 202b are electrically connected, via a circuit substrate disposed in the measurement apparatus via the electrode pads 206 and a connector part 406, to a control unit 401 disposed on the circuit substrate.

The voltage applying unit 402 is controlled by the control unit 401, and applies a predetermined voltage (constant voltage) and/or a sweep voltage. Further, the response (typically, current generated over time) is measured by a measuring unit 403 controlled by the control unit 401.

Note that although this measurement apparatus includes the measuring unit 403 and the voltage applying unit 402 independently, the embodiment of the present invention is not limited to the above and the measuring unit and the voltage applying unit may be integrated.

The control unit 401 is a processor. Examples of the control unit 401 include, but not limited to, a central processing unit (CPU), a microprocessor, a processor core, a multiprocessor, an ASIC (application-specific integrated circuit), an FPGA(field programmable gate array), and a GPU (Graphics Processing Unit).

The control unit 401 reads a program stored in a storage unit 404, and controls the voltage applying unit 402, the measuring unit 403, the storage unit 404, and an analysis output unit 405 in accordance with this program, thereby executing predetermined arithmetic processing. In other words, the measurement method described below is executed.

Further, the control unit 401 is capable of appropriately writing/reading the arithmetic result according to the program to/from the storage unit 404.

The storage function of the storage unit 404 is realized by, for example, a non-volatile memory such as an HDD (hard disk drive) and an SSD (solid-state drive). Further, the storage unit 404 may have a function as a memory for writing or reading the progress of the arithmetic processing by the control unit 401. The memory function of the storage unit 404 is realized by a volatile memory such as a RAM (random access memory) and a DRAM (dynamic random access memory). Typically the control unit 401, the storage unit 404, and the like constitute a computer.

The measuring unit 403 is controlled by the control unit 401, and measures the response when a voltage is applied.

Further, the analysis output unit 405 is a function realized by the program stored in the storage unit 404 being executed by the control unit 401. The analysis output unit 405 applies the substrate used for measurement and the obtained response to a classifier stored in the storage unit, and outputs microbiota information regarding a specimen.

### [Operation of this measurement apparatus]

Next, the operation of this measurement apparatus 100 will be described.

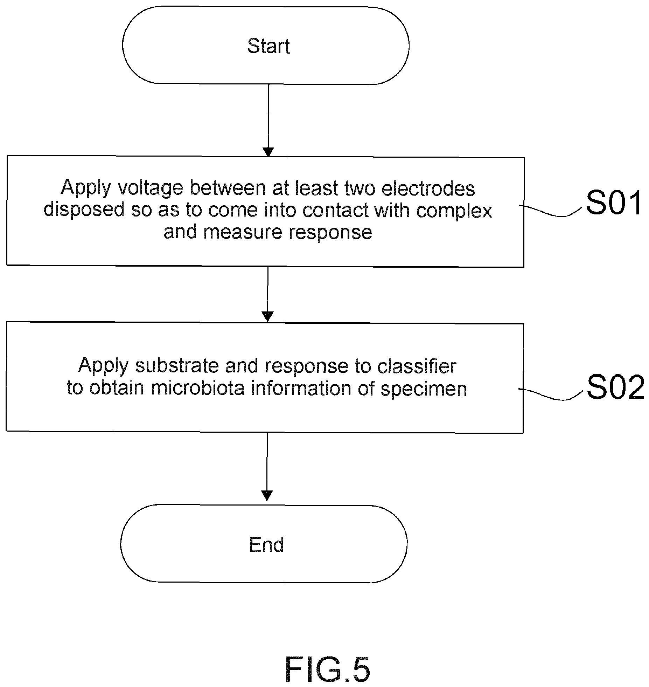

This measurement apparatus 100 operates in accordance with a program as follows. Fig. 5 is a flowchart in which the control unit 401 of the measurement apparatus 100 performs measurement in accordance with a program stored in the storage unit 404. In other words, Fig. 5 is a flowchart of a measurement method executed using this measurement apparatus.

Typically, the operation described above is started when the measurement apparatus 100 receives an instruction to start measurement by a user by, for example, operating the button 103. At this time, the measurement target is typically a biosensor, in which a complex has been introduced, which has been prepared by a user in advance. That is, a biosensor in which a complex has been introduced is prepared by a user before starting measurement.

In the preparation of a biosensor in which a complex has been introduced, a complex may be prepared outside the cell of the biosensor and then introduced in the cell, or a specimen or the like may be sequentially introduced and a complex may be prepared in the cell.

In the case where a complex is prepared outside the cell, typically, a method of causing a specimen containing a microorganism to come into contact with (typically, be mixed with) a medium containing a substrate a composite can be used although not particularly limited. In the case where a complex is prepared in the cell, typically, a method of introducing a medium containing a substrate (or containing no substrate) in the cell, introducing a specimen thereinto (or introducing a substrate and a specimen), and mixing them can be used.

Examples of the substrate include, but not particularly limited to, a carbon source compound, a nitrogen source compound, an inorganic salt, and a mixture thereof.

The content of the substrate in the complex is not particularly limited, but is typically 0.001 to 10 mass% with respect to the total mass of the composite. Note that the composite may contain one type of a substrate or two or more types of substrates. In the case where the composite contains two or more types of substrates, the total content thereof is favorably within the numerical value range described above.

Examples of the carbon source compound include a sugar or a sugar alcohol such as glucose, fructose, sucrose, mannose, maltose, mannitol, xylose, arabinose, galactose, starch, molasses, sorbitol, and glycerin; an organic acid such as acetic acid, citric acid, lactic acid, fumaric acid, maleic acid, and gluconic acid; and an alcohol such as methanol, ethanol, and propanol.

Examples of the nitrogen source compound include an inorganic or organic ammonium compound such as ammonium chloride, ammonium sulphate, ammonium nitrate, and ammonium acetate; urea, ammonia water, sodium nitrate, and potassium nitrate.

Examples of the inorganic salt include primary potassium phosphate, tertiary potassium phosphate, magnesium sulfate, sodium chloride, ferrous nitrate, manganese sulfate, zinc sulphate, cobalt sulfate, and calcium carbonate.

Examples of the mixture include meat extract, peptone, polypeptone, yeast extract, dried yeast, corn steep liquor, skim milk powder, defatted soybean hydrochloric acid hydrolysate, and extract of animals and plants or microbial cells.

Further, in addition to the above, for example, biotin, thiamine (vitamin B1), pyridoxine (vitamin B6), pantothenic acid, inositol, and nicotinic acid can be used as vitamins.

The medium favorably contains water. The pH of the medium is not particularly limited, but is generally favorably 6 to 8.

With reference to Fig. 5 again, when a biosensor in which a complex has been introduced is prepared and inserted into the insertion port 104 of the measurement apparatus 100 and the measurement apparatus 100 receives an instruction to start measurement, the control unit 401 controls the voltage applying unit 402 to apply a voltage between at least two electrodes disposed so as to come into contact with the complex and controls the measuring unit 403 to measure a response (Step S01).

At this time, the voltage to be applied may be a specific voltage (constant voltage) or a sweep voltage. The response is a change in the generated current with respect to the voltage application time.

A specific example of the response described above will be described by taking the case where a specimen is saliva and the saliva contains PG (Porphyromonas gingivalis) bacteria, AA (Actinobacillus actinomycetemcomitans) bacteria, or the like as an example.

Note that the PG bacteria and the AA bacteria are known to be the causative bacteria of periodontal disease (referred to also as "periodontal disease bacteria" in the present specification). In the case where the microbiota in the saliva is investigated and it is speculated that the bacteria described above are present or predominant, it is useful in that it can be an important index for the treatment decision of periodontal disease.

This measurement apparatus is capable of acquiring microbiota information regarding a specimen containing various microorganisms described below, and particularly excellent effects are easily achieved when the specimen contains saliva.

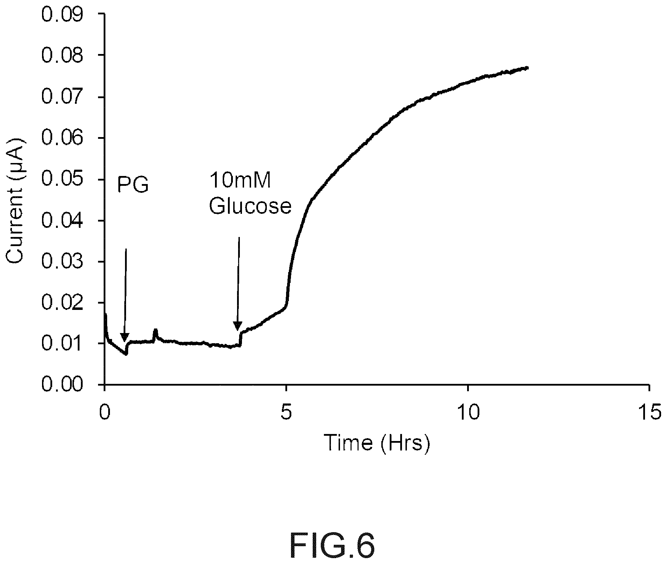

Fig. 6 shows a response of a solution containing a specimen that contains PG bacteria. The horizontal taxis represents the voltage application time (unit: hour), and the vertical axis represents the generated current (unit: µA).

It can be seen that in the case where a medium is only water to which a substrate is not added, the current value does not increase over time. That is, it can be seen that the PG bacteria do not generate a current. Meanwhile, it can be seen that when glucose is added to the medium (content of 10 mM in the solution), the generated current increases rapidly.

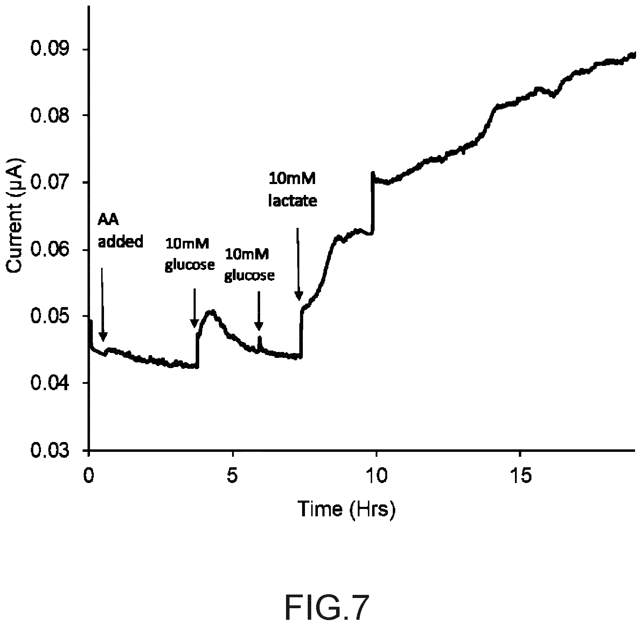

Meanwhile, Fig. 7 shows a response of a solution containing a specimen that contains AA bacteria. In the case of AA bacteria, it can be seen that when glucose is added to the solution, the current value increases temporarily, but the current value does not increase over time. Further, it can be seen that in the case where glucose is added for the second time, substantially no temporary increase in the current value is observed and the response is different from that at the time of the first glucose addition.

Meanwhile, in the case of adding lactic acid, it can be seen that the generated current rapidly increases. That is, it can be seen that the AA bacteria specifically generate a current in the presence of lactic acid.

As described above, it has been found by the examination by the present inventors for the first time that the expression status (i.e., a response to be obtained, particularly, electrochemical response) of the current generation ability of each bacterium differs depending on the microbiota of a specimen and the type and concentration of a substrate present in the solution, and thus, the present invention has been completed.

Next, with reference to Fig. 5 again, the control unit 401 controls the analysis output unit 405 to apply a substrate and a response to a classifier (estimation model), thereby acquiring microbiota information regarding a specimen (Step S02).

In this step, the response and substrate described above are applied to the classifier. The classifier is typically a learned neural network. The classifier described above is a classifier which has been pre-learned by using learning data including known microbiota information regarding a learning specimen, a substrate, and a response to be obtained so as to output microbiota information when the substrate (typically, a type of the substrate and/or concentration of the substrate in a solution) and the response (typically, a change in the current value over time) are input.

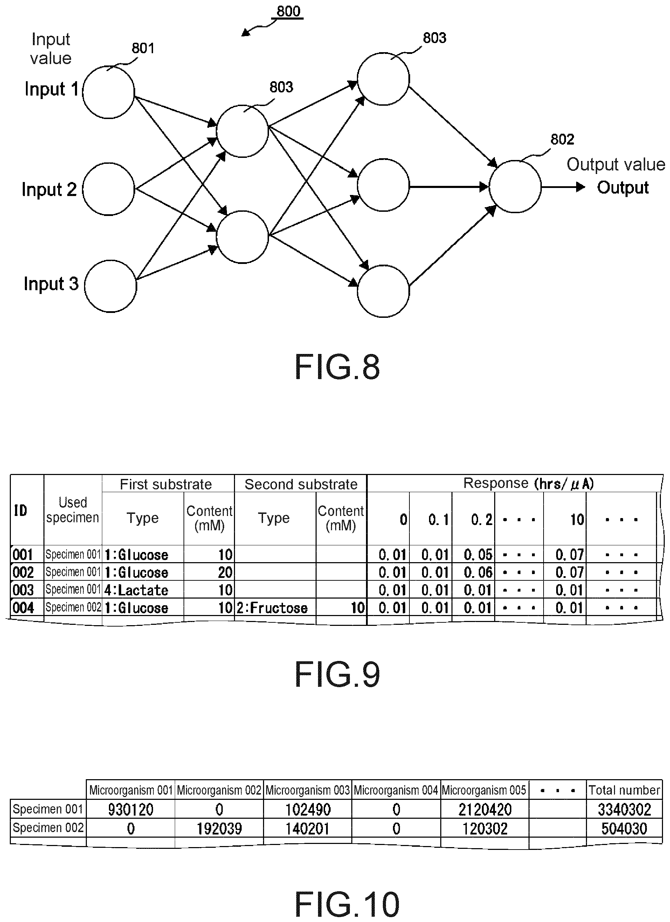

An example of a neural network that can be applied to this measurement apparatus will be described with reference to the drawings. Fig. 8 is a diagram showing an example of a four-layer deep neural network. A four-layer deep neural network (hereinafter, referred to also as "DNN".) 800 shown in Fig. 8 includes, as an input layer, three nodes 801 corresponding to input values Input1, Input2, and Input3, and, as an output, one node 802 corresponding to an output value Output.

The four-layer DNN includes an intermediate layer including two layers, and each of nodes 803 of the intermediate layer has a weight. The four-layer DNN is capable of generating an appropriate weight of each node by using a large amount of input/output data. This is generally called deep learning.

Here, the data to be learned by the neural network is an n-dimensional tensor data structure. Fig. 9 and Fig. 10 shows an example of learning data (training data) to be learned by the neural network. Fig. 9 shows the relationship between microbiota information, a substrate, and a response (change in the current value over time) regarding a learning specimen whose microbiota information is known, which is the data prepared for learning in advance.

Note that the microbiota information (typically, fungal flora information) includes at least the type of a microorganism contained in a specimen, and is favorably information that includes the type and amount of the microorganism (in other words, the amount of a microorganism for each type). The information including the type and amount of a microorganism is not particularly limited. Examples thereof include information acquired by the method described in Japanese Patent Application Laid-open No. 2008-206516, Japanese Patent Application Laid-open No. 2017-23093, or the like.

That is, the learning specimen is favorably a specimen with the type and amount of a microorganism known by a method using a DNA chip, an invader method, a quantitative PCR method (q-PCR) such as a real-time PCR (Polymerase Chain Reaction) method, or the like.

The learning data includes a response (typically, an electrochemical response) obtained using a predetermined substrate regarding the known (microbiota information) learning specimen described above. Here, the fact that the learning data "includes known microbiota information regarding a learning specimen, a substrate, and a response to be obtained" means that it is a set of data of the response obtained when measurement was performed using this measurement apparatus regarding a learning specimen having predetermined microbiota information, by using a medium containing a predetermined amount of a predetermined type of substrate.

With reference to Fig. 9 and Fig. 10 again, a specific example of learning data will be described. First, in Fig. 9, a unique number is given to each learning specimen ("specimen 001" and "specimen 002"). Further, the electrochemical response corresponding to the type of the used substrate and the content (mM) in the solution is identified by "ID" indicating the individual measurement result.

In Fig. 7, for example, a measurement result ID "004" indicates that glucose and fructose have been mixed and used as a substrate for the specimen 002. Note that information regarding a third substrate and subsequent substrates may be provided.

Fig. 10 shows microbiota information regarding each learning specimen. The learning data includes information regarding the amount of a microorganism for each type, which is included in each learning specimen. Note that in Fig. 8, the number of each learning specimen corresponds to the number of a specimen of the data shown in Fig. 9. That is, by performing supervised learning using a plurality of pieces of microbiota information for each specimen shown in Fig. 9 as correct answer data, it is possible to realize a classifier that outputs microbiota information when a substrate (type and/or concentration) and a response are applied.

The microbiota information acquired in this step includes at least information regarding a type of a microorganism contained in a specimen, and favorably includes information regarding the type and amount of a microorganism (in other words, amount of a microorganism for each type). In the case where the amount of a microorganism for each type is included, it is also possible to compare the measurement results with each other over time and acquire information for determining whether or not there is an abnormality.

For example, in the case where the specimen is saliva, by acquiring a specimen a plurality of times from the same subject over a total of several days and analyzing the difference between the specimens (e.g., a change in the amount of a specific microorganism), it is possible to detect a change in a specific microorganism in the oral cavity of the subject over time, e.g., the fact that the number of caries bacteria is increasing.

### [Processing sequence]

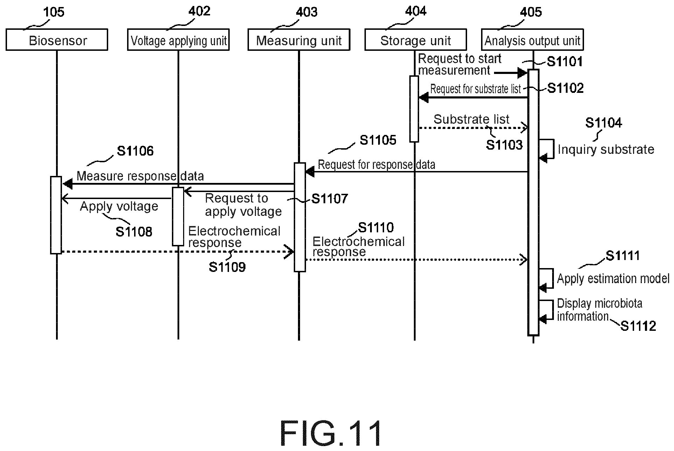

Fig. 11 is a sequence diagram showing more specific processing according to an embodiment, which is executed in this measurement apparatus. The sequence in Fig. 11 will be described as being executed in the state where a solution containing a specimen and a substrate is introduced in the cell 201 of the biosensor 105, the biosensor 105 is inserted into the insertion port 104 of the measurement apparatus, and the biosensor 105 is electrically connected to the connector part 406.

Note that in the following description, the operations of the respective units of the measurement apparatus 100 are controlled by the control unit 401 that operates in accordance with a program stored in the storage unit 404, and executed.

First, the analysis output unit 405 receives a request to start measurement from an operator (S1101). Next, the analysis output unit 405 requests for a substrate list to the storage unit 404 (S1102).

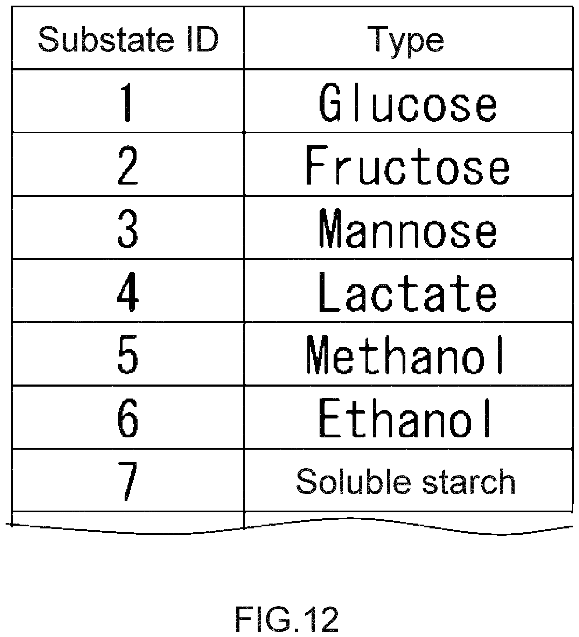

Here, the substrate list is a list (no duplication) of substrates which has been used in the learning data of the classifier described above (Fig. 9), and stored in the storage unit 404. Fig. 12 shows an example of the substrate list.

Next, the storage unit 404 passes the substrate list to the analysis output unit 405 (S1103). The analysis output unit 405 that has received the substrate list executes processing for inquiring the type of a substrate to be used in measurement and the content in the solution (S1104: substrate inquiry).

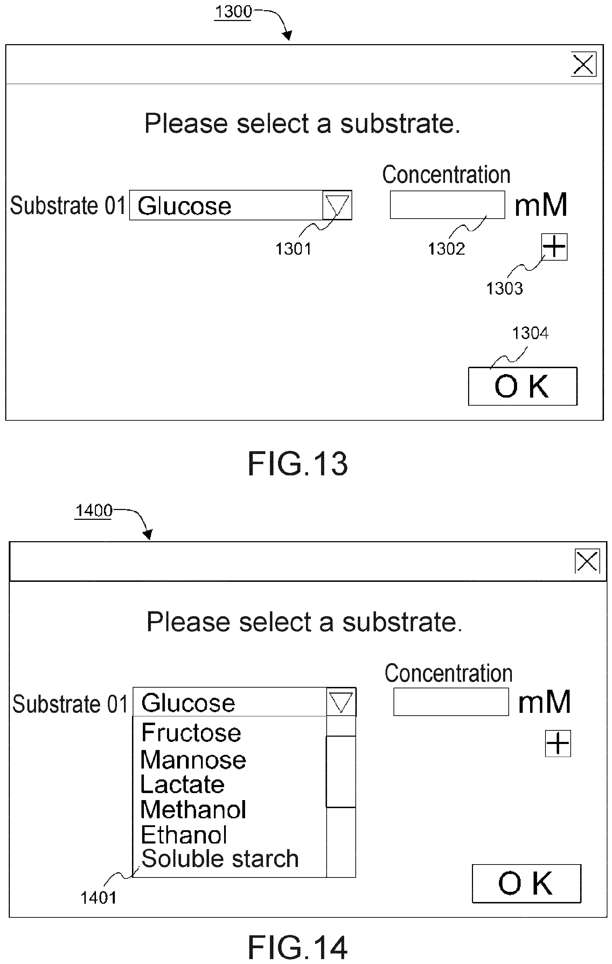

The substrate inquiry processing is performed by, for example, displaying the substrate inquiry screen shown in Fig. 13 on the display unit 102 by the analysis output unit 405. Note that in the following description, the transition or the like of each display screen is executed by the analysis output unit 405.

Fig. 13 shows an example of a substrate inquiry screen display. An operator can input a substrate to be used in measurement, via a GUI using a substrate inquiry screen display 1300 displayed on a display unit 107.

At this time, the substrate to be input is favorably selected from the substrate list described above from the viewpoint of making it easier to apply the obtained measurement result to the classifier and making it possible to acquire more accurate microbiota information.

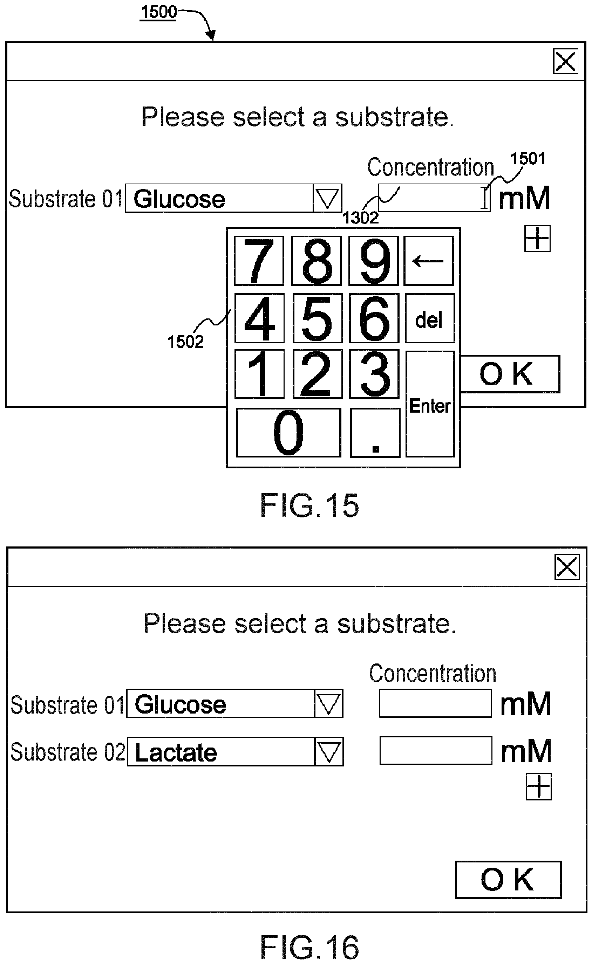

In the substrate inquiry screen display 1300, when an operator operates a pull-down button 1301, the display transitions to the screen display shown in Fig. 15. In the substrate inquiry screen display 1200, a pull-down list 1401 is displayed, and the operator can select a substrate to be used in measurement, via the GUI using the screen described above.

The substrates displayed in this list are the same as the types of the substrates stored in the substrate list described above.

That is, the analysis output unit 405 determines, on the basis of the substrate list acquired from the storage unit 404, the type of the substrate to be displayed on the pull-down list 1401. Typically, the substrate described in the substrate list is displayed on the pull-down list 1401.

Next, in the case where the operator inputs the concentration of the substrate, when he/she selects (typically, touches) a substrate concentration box 1302, the display transitions to the screen display shown in Fig. 15. In a substrate inquiry screen display 1500, a cursor 1501 is displayed on the substrate concentration box 1302 and a numeric key 1502 for inputting a numerical value is displayed. The operator can input the concentration of the substrate by operating (typically, touching) the numeric key 1502 described above. In accordance with the GUI described above, the operator can simply input the concentration of the substrate not via an input device for a keyboard. For this reason, it is possible to miniaturize and simplify the measurement apparatus, and more simply acquire microbiota information on-site.

Further, in the case where a plurality of substrates is used in measurement, when the operator operates (typically, touches) an add button 1303, the display transitions to the screen display shown in Fig. 16 and a plurality of substrates can be input. In accordance with the GUI described above, even in the case where a plurality of substrates is used, the substrates can be more easily input.

When the input is finished, the operator operates a button 1304, and the analysis output unit 405 acquires information regarding the type and concentration of a substrate to be used in measurement.

Next, with reference to Fig. 11 again, the analysis output unit 405 requests for electrochemical response data to the measuring unit 403 (S1105). When receiving the request for electrochemical response data, the measuring unit 403 controls the biosensor 105 to start measurement of electrochemical response data (S1106). At this time, the measuring unit 403 requests the voltage applying unit 402 to apply a voltage between electrodes of the biosensor (S1107).

The voltage applying unit 402 that has received, from the measuring unit, a request to apply a voltage, applies a predetermined voltage and/or a sweep voltage between electrodes of the biosensor (S1108).

Note that the steps described above (S1106 to S1108) can be made simpler in the case where the measuring unit and the voltage applying unit are integrated. For example, a voltage applying/measuring unit that has received a request for response data only needs to apply a voltage to the biosensor and measure the response data.

The biosensor 105 passes the electrochemical response, i.e., the measurement result to the measuring unit 403 (S1109). After the measurement is finished, the measuring unit 403 passes the obtained electrochemical response to the analysis output unit 405 (S1110).

The analysis output unit 405 that has acquired the electrochemical response applies the type and concentration of the substrate acquired by the substrate inquiry (S1104) and the electrochemical response acquired from the measuring unit 403 to the classifier to acquire microbiota information (S1111).

The analysis output unit 405 that has acquired microbiota information displays the microbiota information on the display unit 102 (S1112).

As described above, in accordance with this measurement apparatus, it is possible to easily output microbiota information regarding a specimen.

Note that although the case where the specimen is saliva and the saliva contains the PG bacteria, the AA bacteria, or the like has been described above as an example, the specimen that can be a target of measurement using the measurement apparatus according to the embodiment of the present invention is not limited to the above and is not particularly limited as long as the specimen contains a microorganism, and the present invention is applicable also to any known specimen. In particular, the present invention has the great advantage of being applicable to a specimen containing a microorganism and a solvent without the need for pretreatment.

In particular, in the case where a specimen is saliva and the saliva contains periodontal disease bacteria such as the PG bacteria and the AA bacteria, favorably, information for diagnosing the oral health condition of the subject who has provided the saliva can be easily acquired from the microbiota information acquired by this measurement apparatus.

AS the specimen, for example, body fluid of animals can be used in addition to saliva. Examples of the animals include, but particularly not limited to, a human and livestock.

Examples of the body fluid include blood, lymph, tissue fluid, body cavity fluid, digestive fluid, sweat, tears, snot, urine, semen, vaginal fluid, amniotic fluid, and milk.

By acquiring microbiota information regarding the body fluid described above, it is possible to use the microbiota information as information for diagnosing and preventing various illnesses.

Further, examples of the specimen include also liquid containing sludge and water and sewerage. Examples of the liquid containing sludge include liquid containing activated sludge used for water treatment and liquid containing sludge generated by waste treatment.

By acquiring microbiota information regarding these specimens, for example, it is possible to use the microbiota information as information for more efficiently performing operation management of water treatment plants.

Further, as the specimen, biological fluid of a plant, environmental water, or the like may be used.

Examples of the biological fluid of a plant include, but not particularly limited to, conduit fluid, sieve tube fluid, petiole juice, and leaf blade juice. By acquiring microbiota information regarding these specimens, for example, it is possible to use the microbiota information as information for diagnosing and preventing plant diseases.

Further, examples of the environmental water include river water and groundwater. By acquiring microbiota information regarding these specimens, it is possible to use the microbiota information as information for predicting the impact on plants when using the water as agricultural water and for more efficiently cultivating plants.

Further, examples of the target microorganism include, but not particularly limited to, microorganisms registered in DDBJ (DNA Data Bank of Japan), EMBL (European Molecular Biology Laboratory) Nucleotide Sequence Database, GenBank, or the like.

The microorganism may be a microorganism of the genus Porphyromonas, the genus Tannerella, the genus Treponema, the genus Campylobacter, the genus Fusobacterium, the genus Parvimonas, the genus Streptococcus, the genus Aggregatibacter, the genus Capnocytophaga, the genus Eikenella, the genus Actinomyces, the genus Veillonella, the genus Selenomonas, the genus Lactobacillus, the genus Pseudomonas, the genus Haemophilus, the genus Klebsiella, the genus Serratia, the genus Moraxella, or the genus Candida.

Further, the microorganism may be Porphyromonas gingivalis, Tannerella forsythia, Treponema denticola, Prevotella intermedia, Aggregatibacter actinomycetemcomitans, Fusobacterium nucleatum subsp. vincentii, Fusobacterium nucleatum subsp. polymorphum, Fusobacterium nucleatum subsp. animalis, Fusobacterium nucleatum subsp. nucleatum, Streptococcus mutans, Streptococcus salivarius, Streptococcus sanguis, Streptococcus miris, Actinomyces viscosus, Lactobacillus gasseri, Lactobacillus phage, Lactobacillus casei, Lactobacillus delbrueckii subsp. bulgaricus, Streptococcus aureus, Pseudomonas aeruginosa, Streptococcus pyogenes, Streptococcus pneumoniae, Haemophilus influenzae, Klebsiella pneumoniae, Serratia marcescens, Serratia macescens, Moraxella catarrhalis, Candida albicans, Campylobacter gracilis, Campylobacter rectus, Campylobacter showae, Fusobacterium periodonticum, Parvimonas micra, Prevotella nigrescens, Streptococcus constellatus, Campylobacter concisus, Capnocytophaga gingivalis, Capnocytophaga ochracea, Capnocytophaga sputigena, Eikenella corrodens, Streptococcus gordonii, Streptococcus intermedius, Streptococcus mitis bv 2, Actinomyces odontolyticus, Veillonella parvula, Actinomyces naeslundii II, Selenomonas noxia, or the like.

### [Other embodiments of biosensor]

The biosensor used in the measurement apparatus according to the embodiment of the present invention may be a biosensor that includes, in a cell, a medium that is a solid electrolyte disposed so as to come into contact with an electrode, in which a specimen come into contact with the surface of the medium, thereby forming a complex. Note that in the following description, description of portions similar to those in the measurement apparatus described above is omitted.

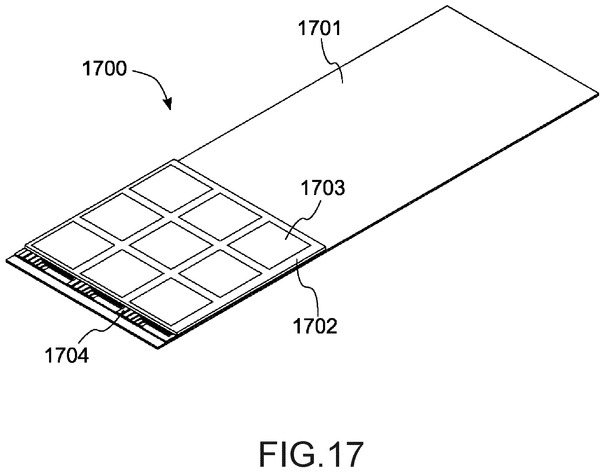

Fig. 17 is a perspective view of a biosensor 1700 that is loaded in this measurement apparatus for use. The biosensor 1700 includes a solid electrolyte 1703 disposed on a support 1701. The biosensor 1700 includes a plurality of solid electrolytes 1703, and each of the solid electrolytes 1703 is disposed in a certain region defined by a frame 1702 and constitutes a plurality of cells together with a pair of electrode described below.

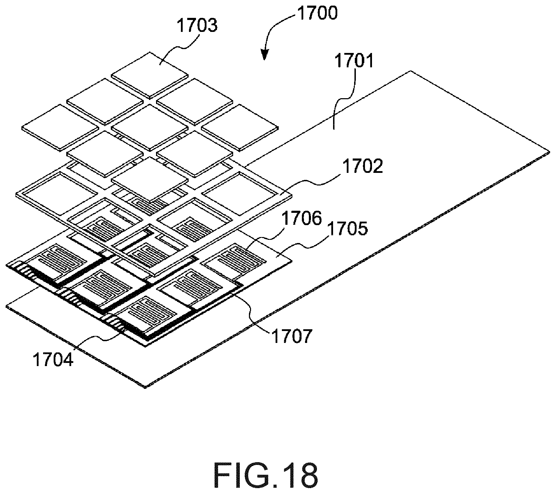

Fig. 18 is an exploded perspective view of the biosensor 1700. An electrode substrate 1705 is disposed between the solid electrolyte 1703 and the support 1701, and a plurality of pairs of electrodes 1706 is disposed on the electrode substrate 1705, each of the pairs of electrodes 1706 being disposed so as to correspond to the solid electrolyte 1703 and come into contact with the corresponding solid electrolyte 1703.

The pair of electrodes 1706 is electrically connected to an electrode pad 1704 by a drawer wiring 1707, and the electrode pad 1704 is configured to be electrically connectable to a circuit substrate disposed in the measurement apparatus via a connector part in the measurement apparatus by being inserted into the body 101.

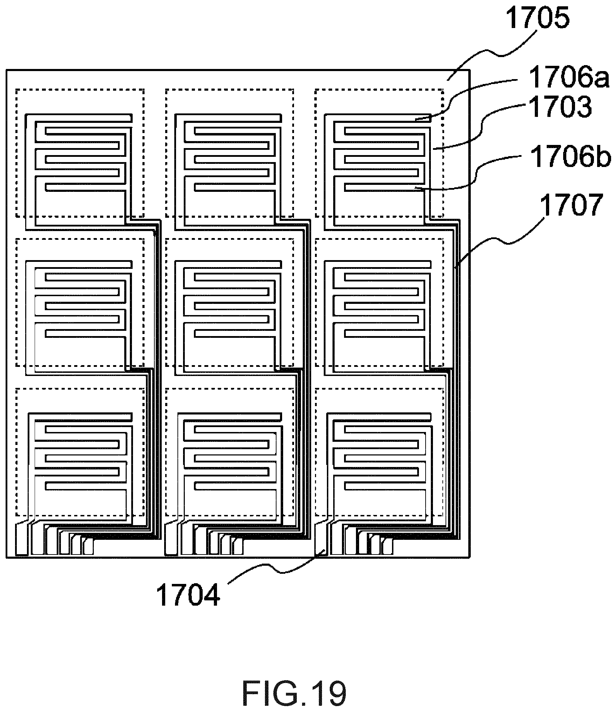

Fig. 19 is a top view of the electrode substrate 1705. On the electrode substrate 1705, a pair of electrodes (comb electrodes including a first electrode 1706a and a second electrode 1706b) disposed so as to correspond to the solid electrolyte 1703 (outline thereof is shown by a broken line in Fig. 19) and come into contact with the corresponding solid electrolyte 1703 is provided. Each electrode is electrically connected to the electrode pad 1704 by the drawer wiring 1707.

A specimen containing a microorganism is caused to come into contact with each electrode (typically, a specimen is added dropwise on each electrode). Next, the solid electrolyte 1703 fixed to the frame 1702 covers thereon, and thus, the electrode, the specimen, and the solid electrolyte come into contact with each other to simultaneously form a plurality of composites. Since the electrode is disposed so as to correspond to the corresponding solid electrolyte in the biosensor 1700, it is possible to simultaneously or sequentially measure a plurality of electrochemical responses regarding the same specimen.

Further, in the case where the solid electrolyte described below contains different substrates, i.e., substrates of different types and/or concentrations, it is possible to eliminate the trouble of the operator changing a medium and simply acquire many more electrochemical responses. As a result, more accurate microbiota information can be acquired.

### (Solid electrolyte)

The solid electrolyte is a medium containing a substrate, and means an electrolyte that can contain a component, which may be contained in the medium described above, and is solid at normal temperature. Typical examples of the form of the solid electrolyte include, but not particularly limited to, a form of hydrogel. Examples of the hydrogel include agar gel and gelatin gel.

The electrolyte described above is favorably one having high ionic conductivity, and more specifically, one in which ions (e.g., hydrogen ions or sulfate ions) are movable.

The biosensor of this measurement apparatus includes a plurality of solid electrolytes, and the substrates contained in the solid electrolytes that are media may be the same or different from each other. In the case where (type and/or concentration of) the substrates contained in the solid electrolytes differ, it is possible to simply acquire electrochemical responses using a large number of substrates according to the same specimen. As a result, more accurate microbiota information can be acquired.

Further, in the case where the substrate contained in the solid electrolyte described above is a substrate included in the learning data described above, more accurate microbiota information is easily acquired.

Note that the fact that the "substrate is included in the learning data" means that the type (favorably, the combination of the type and concentration of the substrate) of the substrate contained in the solid electrolyte is included in the type (favorably, the combination of the type and concentration of the substrate) of the substrate used in the learning data.

For example, in Fig. 9 showing learning data, the measurement result of the ID "001" is the result obtained using 10 mM of glucose as a substrate. In this case, at least one of the solid electrolytes favorably contains glucose.

Continuing the description, for example, other solid electrolytes favorably contain the same substrates as those of the ID 002, ID 003, and ID 004 (favorably, at the same concentrations).

### Industrial Applicability

As described above, the measurement apparatus according to the present invention is capable of easily acquiring microbiota information. Since microbiota information regarding a specimen can be rapidly acquired without performing complex pretreatment on the specimen, excellent effects can be achieved in the case where on-site microorganism management is necessary. For example, it can be used for management of the progress of various illnesses and checking of effects of drugs.

### Reference Signs List

- 100: :measurement apparatus
- 101: :body
- 102: :display unit
- 103: :operation button
- 104: :insertion port
- 105: :biosensor
- 106: :button
- 107: :display unit
- 108: :electrode
- 201: :cell
- 202: :electrode
- 202a: :first electrode
- 202b: :second electrode
- 203: :introduction port
- 204: :conduit part
- 205: :capillary substrate
- 206: :electrode pad
- 206a: :electrode pad
- 207: :cover
- 208: :support
- 401: :control unit
- 402: :voltage applying unit
- 403: :measuring unit
- 404: :storage unit
- 405: :analysis output unit
- 406: :connector part
- 801: :node
- 802: :node
- 803: :node
- 1200: :substrate inquiry screen display
- 1300: :substrate inquiry screen display
- 1301: :pull-down button
- 1302: :substrate concentration box
- 1303: :add button
- 1304: :button
- 1401: :pull-down list
- 1500: :substrate inquiry screen display
- 1501: :cursor
- 1502: :numeric key
- 1700: :biosensor
- 1701: :support
- 1702: :frame
- 1703: :solid electrolyte
- 1704: :electrode pad
- 1705: :electrode substrate
- 1706: :electrode
- 1706a: :first electrode
- 1706b: :second electrode
- 1707: :drawer wiring

## Claims

1. A measurement apparatus, comprising:
a voltage applying unit that applies a voltage between at least two electrodes disposed so as to come into contact with a complex in which a specimen including a microorganism and a medium including a substrate are in contact with each other;
a measuring unit that measures a response when the voltage is applied;
a storage unit in which a classifier is stored; and
an analysis output unit that applies the substrate and the response to the classifier, and outputs microbiota information regarding the specimen, wherein
the classifier is a classifier which has been pre-learned by using learning data including known microbiota information regarding a learning specimen, a substrate, and a response to be obtained so as to output, when the substrate used for measurement and the obtained response are input, microbiota information regarding a specimen to be measured.

2. The measurement apparatus according to claim 1, wherein
one of the electrodes is at least one selected from the group consisting of a reference electrode and a counter electrode.

3. The measurement apparatus according to claim 1 or 2, wherein
the voltage applying unit applies a predetermined voltage.

4. The measurement apparatus according to claim 1 or 2, wherein
the voltage applying unit applies a sweep voltage.

5. The measurement apparatus according to any one of claims 1 to 4, wherein
the specimen includes saliva.

6. The measurement apparatus according to any one of claims 1 to 5, wherein
the specimen includes periodontal disease bacteria.

7. The measurement apparatus according to any one of claims 1 to 6, wherein
the medium is a solid electrolyte.

8. The measurement apparatus according to claim 7, wherein
the substrate included in the solid electrolyte is the substrate included in the learning data.

9. A measurement method, comprising:
applying a voltage between at least two electrodes disposed so as to come into contact with a complex in which a specimen including a microorganism and a medium including a substrate are in contact with each other, and measuring a response; and
applying the substrate and the response to a classifier, and acquiring microbiota information regarding the specimen, wherein
the classifier is a classifier which has been pre-learned by using learning data including known microbiota information regarding a learning specimen, a substrate, and a response to be obtained so as to output, when the substrate used for measurement and the obtained response are input, microbiota information regarding a specimen to be measured.

10. A program that causes a computer to execute the steps of:
applying a voltage between at least two electrodes disposed so as to come into contact with a complex in which a specimen including a microorganism and a medium including a substrate are in contact with each other, and measuring a response; and
applying the substrate and the response to a classifier, and acquiring microbiota information regarding the specimen, wherein
the classifier is a classifier which has been pre-learned by using learning data including known microbiota information regarding a learning specimen, a substrate, and a response to be obtained so as to output, when the substrate used for measurement and the obtained response are input, information for speculating microbiota of a specimen to be measured.

11. A biosensor, comprising
a support; and
a plurality of cells disposed on the support, wherein
each of the cells includes a solid electrolyte and at least two electrodes disposed so as to come into contact with the solid electrolyte, and
the solid electrolytes include substrates different from each other.
